# EUROPEAN PATENT APPLICATION

(11) **EP 2 360 144 A1**
(43) Date of publication of application: **24.08.2011**
(21) Application number: 11155698.1
(22) Date of filing: 23.02.2011
(51) Int. Cl.: C07C 319/20, C07C 323/58

(54) **Production Method of Methionine**

(30) Priority: 24.02.2010 JP 2010039136
(71) Applicant: SUMITOMO CHEMICAL CO., LTD., Tokyo 104-8260 (JP)
(72) Inventor: Hutagalung, Faber Raymond Ojahan, Ehime 792-0025 (JP); Koizumi, Yoshiyuki, Ehime 792-0025 (JP)
(74) Representative: Duckworth, Timothy John

(57) **Abstract**

The present invention aims to provide a method of producing methionine by hydrolyzing M-hydantoin in the presence of a basic potassium compound, which can effectively prevent corrosion of reaction vessels in both the liquid phase and gaseous phase of a hydrolysis solution even at a higher temperature, and enables a longer period of stable production of methionine. Provided is a method of producing methionine, comprising hydrolyzing 5-(β-methylmercaptoethyl)hydantoin in water in the presence of a basic potassium compound in a reaction system having a potassium concentration of not more than 9 wt% at a temperature of not less than 170°C.

## Description

### Technical Field of the Invention

The present invention relates to a method of producing methionine by hydrolyzing 5-(**β**-methylmercaptoethyl)hydantoin in the presence of a basic potassium compound, which can significantly decrease corrosion of reaction vessels, and enables long-term stable production of methionine.

### Background of the Invention

A method of obtaining methionine by hydrolyzing 5-(**β-**methylmercaptoethyl)hydantoin (hereinafter to be referred to as M-hydantoin) is generally performed in the presence of a basic potassium compound such as potassium carbonate and the like, as shown by the following formula.

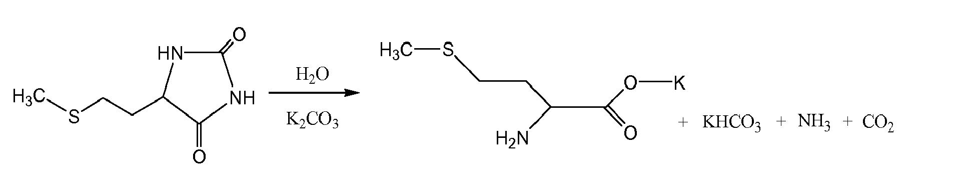

As to the general reaction conditions for this hydrolysis, the pressure is within the range of about 0.5 to 1.5 MPaG and the temperature is within the range of about 150 to 200°C. The corrosion of reaction vessels under such conditions is extremely severe in both the liquid phase part and the gaseous phase part. Reaction vessels made of SUS304L stainless steel are so severely corroded that a 3 mm-thick tray is penetrated in about 3 months under the conditions. In addition, even corrosion resistance of a high-grade austenitic chrome-nickel stainless steel, which has been accepted as more corrosion-resistant, is insufficient under this environment.

### Summary of the Invention

An object of the present invention is to provide a method of producing methionine by hydrolyzing M-hydantoin in the presence of a basic potassium compound, which can effectively prevent corrosion of reaction vessels in both the liquid phase and gaseous phase of a hydrolysis solution even at a higher temperature, and enables a longer period of stable production of methionine.

The present inventors have conducted intensive studies in an attempt to reduce corrosion of hydrolysis reaction vessels even at a higher temperature, and found that when the potassium concentration of the reaction system is not more than a particular level, corrosion of reaction vessels in both the liquid phase and gaseous phase of a hydrolysis solution can be effectively prevented even at a higher temperature, which resulted in the completion of the present invention.

Accordingly, the present invention relates to
[1] a method of producing methionine, comprising hydrolyzing 5-(**β**-methylmercaptoethyl)hydantoin in water in the presence of a basic potassium compound in a reaction system having a potassium concentration of not more than 9 wt% at a temperature of not less than 170°C; and
[2] the method of the above-mentioned [1], wherein the hydrolysis is performed in a reaction vessel having the inner surface made of stainless steel having a Cr element content of 21.0 to 30.0 wt%, a Ni element content of 4.5 to 11.0 wt%, a Mo element content of 1.0 to 5.0 wt%, a N element content of 0.05 to 0.50 wt% and a W element content of not more than 5.0 wt%.

### Effect of the Invention

Using the production method as described in the present invention, methionine can be produced stably for a longer period of time, since the corrosion of reaction vessels is effectively prevented when M-hydantoin is hydrolyzed even at a higher temperature in the presence of a basic potassium compound. Hence, the method has invaluable industrial applicability.

### Description of Embodiments

In the present invention, methionine is obtained in the form of a potassium salt, by hydrolyzing M-hydantoin in water in the presence of a basic potassium compound.

Examples of the basic potassium compound include potassium hydroxide, potassium carbonate, potassium hydrogen carbonate and the like, and two or more kinds thereof can also be used as necessary.

The amount of the basic potassium compound is generally 1 to 10 mol per 1 mol of M-hydantoin, based on potassium.

The hydrolysis is performed in water. The amount of the water is generally 2- to 20-fold weight relative to M-hydantoin.

In addition, the hydrolysis is generally performed using a reaction vessel made of stainless steel.

In the present invention, it is important that the potassium concentration of the reaction system (based on potassium) be maintained at not more than 9 wt%, at which corrosion of the reaction vessel is effectively prevented even at a higher temperature. The above-mentioned potassium concentration is preferably 7.0 to 8.0 wt%.

The reaction temperature of the hydrolysis is not less than 170°C. To shorten the reaction time and reduce the amount of the basic potassium compound, it is preferably not less than 180°C, more preferably 180 to 220°C.

Ammonia and carbon dioxide gas generated during the hydrolysis are recovered and utilized in the M-hydantoin-formation step.

Then, carbon dioxide gas is introduced into the obtained hydrolysis solution to neutralize the solution, whereby methionine is crystallized. The neutralization and crystallization is performed under pressurized condition with carbon dioxide gas, and the precipitated methionine is filtered and separated and, where necessary, washed with water and dried to give methionine as a product.

In the present invention, the reaction vessel to be used for hydrolysis is preferably one having an inner surface made of stainless steel having a Cr element content of 21.0 to 30.0 wt%, a Ni element content of 4.5 to 11.0 wt%, a Mo element content of 1.0 to 5.0 wt%, a N element content of 0.05 to 0.50 wt% and a W element content of not more than 5.0 wt%. In the present invention, the reaction vessel also includes the parts that come into contact with the hydrolysis solution such as auxiliary valves, piping and the like.

When the Cr element content of the above-mentioned stainless steel is less than 21.0 wt%, good corrosion resistance to hydrolysis cannot be performed and, when it exceeds 30.0 wt%, brittleness becomes marked. The Cr element content is preferably 23.5 to 29.5 wt%.

The presence of Ni element is known to decrease the corrosion resistance of stainless steel to hydrolysis. However, a substantial decrease in the corrosion resistance is not observed within the above-mentioned range, and rather, a mechanical property and processability-improving effect is observed. The Ni element content is preferably 5.0 to 8.5 wt%.

When the Mo element content of the above-mentioned stainless steel is less than 1.0 wt%, good corrosion resistance to hydrolysis cannot be performed and, when it exceeds 5.0 wt%, stainless steel shows poor processability and sigma phase embrittlement is promoted. The Mo element content is preferably 1.0 to 4.0 wt%.

When the N element content of the above-mentioned stainless steel is less than 0.05 wt%, good corrosion resistance to hydrolysis cannot be performed and, when it exceeds 0.50 wt%, nitride is precipitated in stainless steel, which will decrease its toughness. The N element content is preferably 0.05 to 0.40 wt%.

When the W element content of the above-mentioned stainless steel exceeds 5.0 wt%, sigma phase is formed to markedly increase brittleness of the stainless steel.

The stainless steel to which the production method of the present invention can be applied is not particularly limited as long as it contains the above-mentioned chemical components. Examples of the commercially available stainless steel containing the above-mentioned components include SUS329J4L, SCS10, UNSS39274, UNSS32808, UNSS32760, UNSS32750, UNSS32707, UNSS32906 and the like, and use thereof is economical.

As for other elements not mentioned above, the presence thereof is not limited as long as the corrosion resistance to hydrolysis at a higher temperature is not markedly impaired, and examples thereof include C element, Si element, Mn element, P element, S element, Cu element and the like. Preferable contents thereof are: C element content not more than 0.03 wt% , Si element content not more than 0.80 wt%, Mn element content not more than 1.10 wt%, P element content not more than 0.03 wt%, S element content not more than 0.03 wt%, and Cu element content not more than 1.00 wt%.

In the present invention, Cu element is hardly contained in the above-mentioned stainless steel. That is, its content may be limited to an ultratrace amount, for example, not more than 1.00 wt%.

### Examples

The present invention is explained in more detail in the following by referring to Examples, whereas the Examples are merely embodiments, and the present invention is not limited thereby. In the Examples, the chemical components of alloy A (stainless steel) were measured by a fluorescence X-ray analyzer.

### Example 1

A hydrolysis supply solution (potassium concentration: 7.5 wt%), which is a mixture of 5-(**β-**methylmercaptoethyl)hydantoin and potassium carbonate, was continuously supplied from the top of an autoclave (supply rate 700 g/hr), and hydrolysis was performed while maintaining pressure 1.0 MPaG and temperature 180°C, during which a reaction product containing a methionine potassium salt was taken out from the bottom part of the autoclave and the generated gas was released from the top of the autoclave. Alloy A shown in Table 1 (composition shown in Table 1, the rest is mostly Fe) was inserted in the liquid phase of the reaction system and maintained for 4 hr to perform a corrosion test.

The results of the corrosion test were obtained by calculating corrosion rate (thickness reduction per year) from the corrosion level measured (decrease in test piece weight per unit time and unit area). The results are shown in Table 1.

### Example 2

In the same manner as in Example 1 except that the pressure was changed to 2.4 MPaG, and the hydrolysis temperature was changed to 220°C, the corrosion test was performed. The results are shown in Table 1.

### Comparative Example

In the same manner as in Example 1 except that the potassium concentration was changed to 10 wt%, the corrosion test was performed. The results are shown in Table 1.

**Table 1**

| | alloy | tempe-rature (°C) | K concen-tration (wt%) | chemical components (wt%) | | | | | corrosion rate (mm/year) |
|---|---|---|---|---|---|---|---|---|---|
| | | | | Cr | Ni | Mo | N | w | |
| Ex. 1 | alloy A | 180 | 7.5 | 24.92 | 7.25 | 3.08 | 0.15 | 0.26 | 4.16 |
| Ex. 2 | alloy A | 220 | 7.5 | 24.92 | 7.25 | 3.08 | 0.15 | 0.26 | 4.28 |
| Comp. Ex. | alloy A | 180 | 10 | 24.92 | 7.25 | 3.08 | 0.15 | 0.26 | 10.31 |

From Table 1, it is appreciated that when the potassium concentration of the reaction system is not more than 9 wt%, the corrosion rate is of the same level as 180°C even when the hydrolysis temperature is 220°C.

### Industrial Applicability

Using the production method as described in the present invention, methionine can be produced stably for a longer period of time, since the corrosion of reaction vessels is effectively prevented when M-hydantoin is hydrolyzed even at a higher temperature in the presence of a basic potassium compound. Hence, the method has invaluable industrial applicability.

## Claims

1. A method of producing methionine, comprising hydrolyzing 5-(**β**-methylmercaptoethyl)hydantoin in water in the presence of one or more basic potassium compounds in a reaction system having a potassium concentration of not more than 9 wt% at a temperature of not less than 170°C.

2. A method according to claim 1, wherein the hydrolysis is performed in a reaction vessel having the inner surface made of stainless steel.

3. A method according to claim 2, wherein the stainless steel has a Cr element content of 21.0 to 30.0 wt%, a Ni element content of 4.5 to 11.0 wt%, a Mo element content of 1.0 to 5.0 wt%, a N element content of 0.05 to 0.50 wt% and a W element content of not more than 5.0 wt%.

4. A method according to claim 3, wherein the stainless steel has a Cr element content of 23.5 to 29.5 wt%, a Ni element content of 5.0 to 8.5 wt%, a Mo element content of 1.0 to 4.0 wt%, a N element content of 0.05 to 0.40 wt% and a W element content of not more than 5.0 wt%.

5. A method according to any one of the preceding claims wherein the basic potassium compound is potassium hydoxide, potassium carbonate, potassium hydrogen carbonate or a mixture thereof.

6. A method according to any one of the preceding claims wherein the reaction temperature is from 180 to 220°C.

7. A method according to any one of the preceding claims wherein the potassium concentration in the reaction system is from 7 to 8 wt%.

8. A method according to any one of the preceding claims wherein carbon dioxide generated is recoved and introduced into the hydrolysis solution to neutralise the solution.
